# EUROPÄISCHE PATENTANMELDUNG

(11) **EP 1 683 530 A1**
(43) Veröffentlichungstag der Anmeldung: **26.07.2006**
(21) Anmeldenummer: 06000618.6
(22) Anmeldetag: 12.01.2006
(51) Int. Cl.: A61L 2/18, A61L 2/26, A61M 25/00

(54) **Aufbewahrungsflüssigkeit und Aufbewahrungsvorrichtung für Katheter**

(30) Priorität: 24.01.2005 DE 202005001092 U; 18.02.2005 DE 202005002654 U
(71) Anmelder: Urovision Gesellschaft für Medizinischen Technologie Transfer mbH, 83043 Bad Aibling (DE)
(72) Erfinder: Jentsch, Peter, 14943 Luckenwalde (DE); Löchner, Dieter, Dr., 82418 Murnau (DE)
(74) Vertreter: Patentanwälte Hofstetter, Schurack & Skora

(57) **Zusammenfassung**

Die vorliegende Erfindung betrifft eine Aufbewahrungsflüssigkeit für Katheter, die eine Kombination aus
(a) einer Desinfektionslösung;
(b) einem Lösungsvermittler;
(c) einer Pufferlösung; und
(d) einem Gleitmittel
umfasst. Die Erfindung betrifft weiterhin eine Aufbewahrungsvorrichtung für Katheter bestehend aus einer wieder verschließbaren Hülle zur Aufnahme des Katheters und einer Aufbewahrungsflüssigkeit.

## Beschreibung

Die vorliegende Erfindung betrifft eine Aufbewahrungsflüssigkeit und eine Aufbewahrungsvorrichtung für einen Katheter.

Aufbewahrungsflüssigkeiten und Aufbewahrungsvorrichtungen für Katheter sind bekannt. Insbesondere bei der Katheterisierung der Harnblase kann es zur Entstehung von Harnwegsinfektionen meist durch Einschleppung von Erregern bei der Katheterisierung der Harnblase bzw. durch ein Aufsteigen der Erreger bei liegendem Blasenkatheter kommen. Besonders groß ist diese Gefahr bei der oft mehrmals am Tag durchzuführenden Einmal-Katheterisierung. Um entsprechende Infektionen zu vermeiden, wird für jede Einmal-Katheterisierung ein neuer, steril verpackter Einmalkatheter verwendet. Neben der hohen Anzahl an verbrauchten Katheter ergibt sich bei bekannten Kathetersets zudem die Notwendigkeit separat Gleitmittel, insbesondere bei der Katheterisierung der männlichen Harnblase, in die Körperöffnung einzuspritzen. Einerseits ergibt sich daher bei der bekannten Einmal-Katheterisierung das Problem eines hohen Katheterverbrauchs und andererseits die Notwendigkeit einer separaten Gleitmittelzugabe, die wiederum ein Infektionsrisiko in sich birgt.

Aufgabe der vorliegenden Erfindung war es daher, eine Aufbewahrungsflüssigkeit und eine Aufbewahrungsvorrichtung für Katheter bereitzustellen, wodurch einerseits der Katheterverbrauch gesenkt werden kann und andererseits eine vereinfachte und schnellere Katheterisierung gewährleistet wird.

Gelöst werden diese Aufgaben durch eine Aufbewahrungsflüssigkeit mit den Merkmalen des Patentanspruchs 1 und eine Aufbewahrungsvorrichtung gemäß den Merkmalen des Patentanspruchs 8.

Vorteilhafte Ausgestaltungen sind in den jeweiligen Unteransprüchen beschrieben.

Eine erfindungsgemäße Aufbewahrungsflüssigkeit umfasst eine Kombination aus einer Desinfektionslösung, einem Lösungsvermittler, einer Pufferlösung und einem Gleitmittel. Durch die erfindungsgemäße Zusammensetzung der Aufbewahrungsflüssigkeit wird einerseits die sterile Aufbewahrung des Katheters gewährleistet. Zudem kann auf die separate Zugabe von Gleitmittel verzichtet werden, da die erfindungsgemäße Zusammensetzung bereits ein geeignetes Gleitmittel umfasst. Dadurch wird eine vereinfachte und schnellere Katheterisierung gewährleistet, das Risiko von Infektionen wird deutlich gesenkt. Des Weiteren können bereits benutzte Katheter erneut mit der Aufbewahrungsflüssigkeit benetzt werden, so dass eine erneute Desinfektion des Katheters und ein entsprechender Gleitmittelauftrag erfolgt. Der Katheter kann somit vorteilhafterweise mehrfach verwendet werden.

In einer vorteilhaften Zusammensetzung enthält die erfindungsgemäße Aufbewahrungsflüssigkeit die genannten Bestandteile in folgenden Mengen:

| | | |
|---|---|---|
| (a) | Desinfektionslösung | 0,1 - 0,5 g, insbesondere 0,3 g; |
| (b) | Lösungsvermittler | 20,0 - 80,0 g, insbesondere 50,0 g; |
| (c) | Pufferlösung | 0,1 - 3 g, insbesondere 0,5 g; |
| (d) | Gleitmittel | 5.0 - 20,0 g, insbesondere 10,0 g; und |
| (e) | destilliertes Wasser | 500,0 - 1500 g, insbesondere 1000 g sowie |

Anteile an EDTA.

In einer weiteren vorteilhaften Zusammensetzung ist bei der erfindungsgemäßen Aufbewahrungsflüssigkeit die Desinfektionslösung Chlorhexidinglukonat oder Chlorhexidindiacetat, der Lösungsvermittler kann Sorbitol sein, als Pufferlösung kann eine Natriumacetat-Lösung und als Gleitmittel ein Polyethylenglycol (Macrogol) mit einer mittleren relativen Molmasse von 200 - 600 (Handelsnamen Macrogol 200 bis Macrogol 600), insbesondere mit einer mittleren relativen Molmasse von 400 verwendet werden.

Eine erfindungsgemäße Aufbewahrungsvorrichtung für einen Katheter besteht aus einer wiederverschließbaren Hülle zur Aufnahme des Katheters und der Aufbewahrungsflüssigkeit nach einer der vorgenannten Zusammensetzungen. Die Hülle kann dabei köcherartig zur Aufnahme eines oder mehrerer Katheter ausgebildet sein. Durch die vorteilhafte Ausgestaltung der Aufbewahrungsvorrichtung ist es möglich, dass ein darin aufbewahrter Katheter mehrfach verwendet werden kann. Dazu ist es lediglich notwendig, den bereits benutzen Katheter nochmals in die Aufbewahrungsvorrichtung mit der Aufbewahrungsflüssigkeit einzuführen, um so eine erneute Desinfektion und ein Benetzen mit dem Gleitmittel durchzuführen. Durch die Möglichkeit der mehrmaligen Verwendung des Katheters wird der Verbrauch an Kathetern und die damit verbundenen Kosten drastisch gesenkt.

## Patentansprüche

1. Aufbewahrungsflüssigkeit für Katheter umfassend eine Kombination aus:
(a) einer Desinfektionslösung;
(b) einem Lösungsvermittler;
(c) einer Pufferlösung; und
(d) einem Gleitmittel.

2. Aufbewahrungsflüssigkeit nach Anspruch 1,
**gekennzeichnet durch** folgende Zusammensetzung:
| | | |
|---|---|---|
| (a) | Desinfektionslösung | 0,1 - 0,5 g; |
| (b) | Lösungsvermittler | 20,0 - 80,0 g; |
| (c) | Pufferlösung | 0,1 - 3 g; |
| (d) | Gleitmittel | 5.0 - 20,0 g; und |
| (e) | destilliertes Wasser | 500,0 - 1500 g sowie |
Anteile an EDTA.

3. Aufbewahrungsflüssigkeit nach Anspruch 2,
**gekennzeichnet durch** folgende Zusammensetzung:
| | | |
|---|---|---|
| (b) | Lösungsvermittler | 50,0 g; |
| (c) | Pufferlösung | 0,5 g; |
| (d) | Gleitmittel | 10,0 g; und |
| (e) | destilliertes Wasser | 1000 g sowie |
Anteile an EDTA.

4. Aufbewahrungsflüssigkeit nach einem der vorhergehenden Ansprüche,
**dadurch gekennzeichnet,**
**dass** die Desinfektionslösung Chlorhexidinglukonat oder Chlorhexidindiacetat ist.

5. Aufbewahrungsflüssigkeit nach einem der vorhergehenden Ansprüche,
**dadurch gekennzeichnet**
**dass** der Lösungsvermittler Sorbitol ist.

6. Aufbewahrungsflüssigkeit nach einem der vorhergehenden Ansprüche,
**dadurch gekennzeichnet**
**dass** die Pufferlösung eine Natriumacetat-Lösung ist.

7. Aufbewahrungsflüssigkeit nach einem der vorhergehenden Ansprüche,
**dadurch gekennzeichnet,**
**dass** das Gleitmittel ein Polyethylenglycol (Macrogol) mit einer mittleren relativen Molmasse von 200 - 600 ist.

8. Aufbewahrungsvorrichtung für einen Katheter bestehend aus einer wiederverschließbaren Hülle zur Aufnahme des Katheters und der Aufbewahrungsflüssigkeit nach einem der Ansprüche 1 bis 7.

9. Aufbewahrungsvorrichtung nach Anspruch 8,
**dadurch gekennzeichnet,**
**dass** die Hülle köcherartig zur Aufnahme eines oder mehrerer Katheter ausgebildet ist.
